# EUROPEAN PATENT APPLICATION

(11) **EP 1 462 114 A1**
(43) Date of publication of application: **29.09.2004**
(21) Application number: 03075908.8
(22) Date of filing: 28.03.2003
(51) Int. Cl.: A61K 39/395, A61P 37/08, G01N 33/53, C07K 14/705, A61K 31/7088, C12N 15/63, A61K 39/35

(54) **Methods and means to suppress symptons of an allergic disease by inhibiting the glucocorticoid-induced tumor necrosis factor receptor (GRIT or TNFRSF18)**

(71) Applicant: Universiteit Utrecht Holding B.V., 3584 CK Utrecht (NL)
(72) Inventor: van Oosterhout, Antonius Josephus Maria, 3573 PJ Utrecht (NL); Lobato-van Esch, Elisabeth Catharina Adriana Maria, 3527 WH Utrecht (NL); Vissers, Joost Lambert Max, 6525 XE Nijmegen (NL)
(74) Representative: Prins, Adrianus Willem, Mr. Ir.

(57) **Abstract**

The invention relates to the field of immunology, more in particular to the field of immune therapy, even more particularly to a method for regulating tolerance to an allergen in a subject and even more specifically, to methods which involve regulation of a glucocorticoid-induced tumor necrosis factor receptor (GITR). The invention provides methods of treating allergic disorder and compositions for use therein.

## Description

The invention relates to the field of immunology, more in particular to the field of immune therapy, even more particularly to a method for regulating tolerance to an allergen in a subject and even more specifically, to methods which involve regulation of a glucocorticoid-induced tumor necrosis factor receptor (GITR). The invention provides methods of treating allergic disorders and compositions for use therein.

It is now well established that regulatory T-lymphocytes can inhibit harmful immunological disorders directed against self or foreign antigens. At least three different subpopulations of regulatory T-cells have been identified: type-1 regulatory T-cells (Tr1 cells), T-helper type-3 cells (Th3 cells) and naturally occurring "professional" CD4⁺CD25⁺ regulatory T-cells (Treg) also called suppressor cells (Roncarolo *et al.,* 2000; Shevach, 2002).

Tr1 cells are characterized by the production of high levels of interleukin 10 (IL10) upon activation and low levels of IL2 and no IL4. Tr1 cells can be generated by repeated antigen stimulation in the presence of IL10, or vitamin D3 and dexamethasone *in vitro.* Generation of Tr1-like cells has also been demonstrated in human lymphocytes by repetitive stimulation (Jonuleit *et al.,* 2000). Passive transfer of Tr1 cells has been shown to inhibit development of colitis, experimental auto-immune encephalomyelitis and Th2-mediated immune responses in mice. Tr1 cells appear to be generated *in vivo* by dendritic cells producing IL10, as demonstrated in a murine model of inhalation tolerance or by immature dendritic cells. Blocking of IL10 or its cell-surface receptor inhibits the immunosuppressive effects of Tr1 cells. However, cell-contact dependent immunosuppressive effects have been described as well ( Jonuleit *et al.,* 2000).

Th3 cells are characterized by the production of high levels of transforming growth factor β (TGFβ) upon activation. Th3 cells are generated by dendritic cells producing TGFβ, as demonstrated in a murine model of oral tolerance or after antigen stimulation in the presence of TGFβ. Blockade of TGFβ inhibits the immunosuppressive effects of Th3 cells on auto-immunity and transplant rejection (Roncarolo *et al.,* 2000) as well as the immunosuppressive effects induced after oral antigen administration (so-called "oral tolerance").

Naturally occurring "professional" CD4⁺CD25⁺ regulatory T-cells (Treg cells), also called suppressor cells (the terms will be used interchangeably herein), are generated in the thymus and are present in the blood and peripheral tissues at a frequency of approximately 5-10% of all peripheral CD4⁺ cells ( Roncarolo *et al.,* 2000; Shevach, 2002). It is now generally accepted that CD4⁺CD25⁺ Treg cells play an important role in the induction and maintenance of peripheral self-tolerance. Passive transfer of these lymphocytes has been shown to suppress autoimmune diabetes, inflammatory bowel disease and transplant rejection in rodents (Roncarolo *et al.,* 2000). Treg cells require activation via the T-cell receptor to exert their regulatory function, however, once activated, their suppressive effects are antigen-non specific indicating that these cells mediate bystander suppression *in vivo* (Thornton *et al.,* 2000). In humans, CD4⁺CD25⁺ Treg cells have also been described (Jonuleit *et al.,* 2001a). All of the functional studies with human CD4⁺CD25⁺ Treg cells confirm what has been described with murine T-cells. They show strongly reduced proliferative responses upon stimulation even in the presence of IL-2 and suppress proliferative responses of CD4⁺CD25⁺ T-cells. Both human and murine CD4⁺CD25⁺ Treg cells are able to produce low levels of the immunosuppressive cytokines IL10 and TGFβ. However, suppression cannot be overcome by addition of neutralizing antibodies to these cytokines or their respective cell-surface receptors ( Jonuleit *et al.,* 2001a). In contrast to Th3 and Tr1 cells, it has been demonstrated that immunosuppression by CD4⁺CD25⁺ "professional" Treg cells is mediated by a cell-contact dependent mechanism (Shevach, 2002). Although naturally occurring Treg cells are thought to be generated in the thymus, other ways to induce these cells appear to exist. Jonuleit and colleagues (Jonuleit *et al.,* 2000) demonstrated that repetitive stimulation of naive T-cells with allogeneic immature dendritic cells induces a population of T cells that resemble naturally occurring CD4⁺CD25⁺ Treg cells since their suppressive phenotype was dependent on cell-contact, antigen non-specific and APC independent.

The expression of CD4 and CD25 by Treg cells cannot completely discriminate between Treg cells and activated T-cells. Although not exclusively expressed on Treg cells, other markers have been used to identify these cells such as CTLA4, CD45RB/CD45RO (mouse/human) and glucocorticoid-induced tumor necrosis factor receptor (GITR).

Glucocorticoid-induced TNF receptor (GITR, also known as TNFRSF18) was recently identified by differential gene expression (McHugh *et al.,* 2002). Ligation of GITR by an activating antibody appeared to inhibit the suppressive effect of Treg cells on T-lymphocyte activation *in vitro* (McHugh *et al.,* 2002; Shimizu *et al.,* 2002). GITR can be ligated by its natural (or endogenous, the terms will be used interchangeably herein) ligand, GITR ligand (GITRL), leading to the activation of the NF-κB pathway in T-cells and prevention of apoptosis. GITRL is a member of the TNF family and encodes a 177 aminoacid polypeptide with a calculated mass of 20 kDa, and with a type 2 transmembrane topology.

The role of each of the different regulatory T-cell subsets (Th3, Tr1, Treg) in allergic diseases is at present largely unknown. In a mouse model of asthma, it has been shown that treatment with heat-killed *Mycobaterium vaccae* can suppress airway eosinophilia which appears to be mediated by CD4⁺CD45RB^{Io} T-cells (Zuany-Amorim *et al.,* 2002). Anti-IL-10 and anti-TGFβ inhibit these suppressive effects of *M. vaccae,* pointing to a role for both Tr1 and Th3 cells but not to a role for naturally occurring Treg cells. In a model of inhalation tolerance, it has been shown that the prevention of asthma manifestations in a mouse model was mediated by IL-10 producing Tr1 cells. Furthermore, we have demonstrated that the beneficial effects of allergen-immunotherapy in a mouse asthma model are mediated by IL-10, pointing to a role for Tr1 cells in allergen immunotherapy. Similarly, bee-venom immunotherapy in humans has been shown to be mediated by IL-10 producing lymphocytes. Suto and colleagues (Suto *et al.,* 2001) have shown that naturally occurring CD4⁺CD25⁺ Treg cells potentiate Th2 responses and inhibited Th1 responses in a model of antigen-induced eosinophil recruitment after transfer of ovalbumin-specific TCR transgenic T-cells.

Murine models of allergic asthma have widely been accepted as a suitable animal model to study the mechanisms underlying this disease. Previously, we have developed a mouse model with immunologic and pathophysiologic features reminiscent of allergic asthma. In this model, Balb/c mice are sensitized with ovalbumin and repeatedly challenged by inhalation of ovalbumin aerosol. This model is characterized by the presence of ovalbumin-specific IgE antibodies in serum, airway eosinophilia and non-specific hyperresponsiveness concomitant with the appearance of Th2-like cells in lung tissue and lung draining (thoracic) lymph nodes. The role of T-helper lymphocytes in these allergic symptoms has been clearly demonstrated by depletion of CD4⁺ T-cells or a specific T-cell subset as well as by transfer of antigen-specific Th2 cells. In addition, several Th2 derived cytokines including IL4, IL5, IL9 and IL13 are implicated in one or more of these asthma-like symptoms whereas treatment with the Th1 derived cytokine IFNγ down-regulates these characteristics. Hence, Th2-lymphocytes play a role in the initiation and progression of allergic asthma in this mouse model.

At present, glucocorticoids are the most effective drugs in the treatment of asthma to reduce the inflammatory component and hyperresponsiveness. However, glucocorticoids are not very selective and affect a whole range of inflammatory and non-inflammatory cells. Thus, there is a strong need for novel (possibly combinatorial) therapeutic strategies that are more selective and induce long-term relief of symptoms.

The present invention discloses that the number of naturally occurring CD4⁺CD25⁺ Treg cells, upon allergen challenge, significantly increases in the lung tissue but not in the draining lymph nodes in a mouse model of allergic asthma (example 1). Moreover, these Treg cells suppress the allergen induced airway hyperresponsiveness (AHR) as demonstrated by the strong potentiation of AHR upon inactivation of Treg cells by ligation of GITR using an activating monoclonal antibody (example 2). In addition, upregulation of serum IgE levels after allergen challenge is strongly potentiated upon inactivation of Treg cells (example 2). The upregulation of AHR and serum IgE levels after treatment with anti-GITR coincides with increased production of Th2 cytokines upon restimulation *in vitro* (example 3).

These data demonstrate that allergen inhalation stimulates the recruitment of Treg cells, preferably at a site where symptoms associated with an allergic disease are present, and that these cells are able to down-regulate said symptoms. The present invention provides methods to increase the suppressor activity and/or number of CD4⁺CD25⁺ Treg cells which are useful for the treatment of allergic disorders for example, asthma. It is clear that such methods are also applicable in the treatment of related immune- or inflammatory disorders, including auto-immune diseases and transplant rejection.

In a first embodiment the invention provides a method for regulating tolerance to an allergen in a subject, comprising providing, in the presence of said allergen, said subject with an inhibitor capable of regulating the activation of a glucocorticoid-induced tumor necrosis factor receptor (GITR). In yet another embodiment the invention provides a method for inducing and/or increasing tolerance to an allergen in a subject, comprising providing, in the presence of said allergen, said subject with an inhibitor capable of at least in part preventing activation of GITR. Or in other words, the invention provides a method to at least in part diminish (or more preferably completely diminish) symptoms associated with the presence of an allergen and thus to diminish symptoms of an allergic disease.

An allergen is typically defined as a compound that is capable of inducing and/or maintaining symptoms indicative of an allergic disease. Typical examples of allergens are dust, pollen, grasses, some medications, proteins and metals.

Examples of allergic diseases which can be treated according to a method of the invention include, but not limited to allergic rhinitis, food allergy, urticaria, atopic dermatitis, allergic conjunctivis, angioedema, asthma and insect sting allergy.

As one of the main representatives of the family of allergic diseases, we will describe asthma in greater detail as representative of the applications of the present invention. Allergic asthma is, amongst others, characterized by reversible airway obstruction, elevated levels of IgE, chronic airway inflammation and airway hyperresponsiveness to bronchospasmogenic stimuli, airway tissue remodeling and mucus hypersecretion. The allergic inflammatory infiltrate in the airway tissue predominantly consists of eosinophils and CD4⁺ T-lymphocytes. It is now widely accepted that type 2 T-helper (Th2) lymphocytes which produce a limited set of cytokines including interleukin-3 (IL3), IL4, IL5, IL9, IL10 and IL13 play an role in the initiation and progression of allergic asthma.

As disclosed herein within the experimental part, the number of naturally occurring CD4⁺CD25⁺ Treg cells, upon allergen challenge, significantly increases in the lung tissue but not in the draining lymph nodes in a mouse model of allergic asthma. Moreover, these Treg cells suppress the allergen induced airway hyperresponsiveness (AHR) as demonstrated by the strong potentiation of AHR upon inactivation of Treg cells by ligation of GITR using an activating monoclonal antibody. In addition, upregulation of serum IgE levels after allergen challenge is strongly potentiated upon inactivation of Treg cells. The upregulation of AHR and serum IgE levels after treatment with anti-GITR coincides with increased production of Th2 cytokines upon restimulation *in vitro.* Hence, by providing an inhibitor capable of at least in part preventing the activation of GITR to a subject in whom an allergen (which presence results in symptoms of an allergic reaction/disease) is present, symptoms associated with said disease are diminished. More in specific for asthma, a method according to the invention results in down regulation of Th2-lymphocye activation and cytokine production leading to suppression of allergen-induced AHR and serum IgE levels and to at least in partial diminishing of symptoms associated with the presence of said allergen in said subject and tolerance to said allergen is induced or increased.

Preferably, said subject is a mammal and even more preferably said mammal is a human being. Hence the invention provides a method to treat an allergic disease/disorder in a human being. A human being suffering from the symptoms induced and/or maintained by an allergen (and hence suffering from an allergic disease) is now treated by providing said human being with an inhibitor capable of at least in part preventing/blocking of GITR activation and this blocking leads to functional disengagement of Treg cells and the subsequent suppression of Th2-mediated allergic disorder.

The term "at least in part preventing activation of GITR" is defined herein in that the symptoms associated with an allergic disease are at least in part diminished, more preferably complete inhibited, preferably resulting at least in an improvement of overall well being.

Preferably, said inhibitor at least in part prevents ligation of GITR by an endogenous GITR-ligand (GITRL). However, now that it is clear that Treg cells (that comprise said GITR) play such an important role in the induction and/or maintenance of an allergic disease it is also well within the scope of the present invention to provide an inhibitor that is capable of inhibiting the downstream signaling pathway of GITR, for example by interfering in the NF-κB pathway. Examples of NF-κB inhibitors are glucocorticoids and non-steroidal anti-inflammatory drugs such as aspirin and CAMP elevating compounds such as beta-adrenoceptor agonists.

Examples of an inhibitor that is capable of (at least in part) preventing ligation of GITR by an endogenous GITRL are an inhibitor of GITR or an inhibitor of GITRL or a compound that at least in part inhibits the expression of GITR or GITRL.

An inhibitor of GITR is typically capable of binding to GITR without activating the receptor and hence such a compound blocks binding of an endogenous GITRL to GITR and hence activation of GITR is prevented, resulting in the presence of immunosuppressive Treg cells. Examples of these kinds of compounds are antagonists of GITR including but not limited to non-activating antibodies or antibody fragments, peptides or (small) synthetic molecules.

An inhibitor of GITRL is typically capable of binding to GITRL in such a way that further binding to GITR is blocked and hence a similar result as described for the inhibitor of GITR is obtained. Examples of these kinds of compounds include, but are not limited to chimeric fusion proteins such as GITR-Fc or antibodies or antibody fragments, peptides or (small) synthetic molecules.

Yet another way to interfere with ligation of GITR by an endogenous GITRL is by interfering with the expression of either GITR or GITRL. When the endogenous GITR ligand is not expressed, binding with GITR will not take place, resulting in immunosuppressive Treg cells and an induction and/or increase in tolerance to an antigen is obtained. Examples of such inhibitors are anti-sense oligonucleotides or small interfering RNA molecules.

Depending on the nature of an inhibitor that is capable of at least in part preventing activation of GITR, said inhibitor can be administered either enterally, parenterally or preferably targeted at the site where the symptoms due to the presence of an allergen are present, i.e. inhalation in case of asthma using targeting devices such as liposomes. Hence, said inhibitor is preferentially provided locally.

In a preferred embodiment, the invention provides a method for inducing and/or increasing tolerance to an allergen in a subject, comprising providing, in the presence of said allergen, said subject with an inhibitor capable of at least in part preventing activation of GITR, wherein said subject is further provided with said allergen. It is clear that it is possible that said allergen is already present in said subject, for example because said allergen (for example pollen) has entered said subject via inhalation. However, it is also possible that an allergen responsible for the induction and/or maintenance of an allergic disease is temporarily not present or no longer present or is present in a too low concentration. In these circumstances it is particularly advantageous to provide said subject with said inhibitor capable of at least in part preventing activation of GITR in combination with said allergen. It is clear that said inhibitor and said allergen can be provided to said subject in the same or different compositions, sequentially or at the same time. A sequential combination can either be first an inhibitor followed by an allergen or first an allergen followed by an inhibitor.

CD4⁺CD25⁺ Treg cells can induce long-lasting anergy and production of IL10 or TGFβ in CD4⁺ CD25- (Dieckmann *et al.,* 2002; Jonuleit *et al.,* 2002). The immunosuppressive effects of these T-cells depends on IL10 or TGFβ and can therefore be classified as Tr1 or Th3 cells. Thus, methods to enhance the immunosuppressive effects of Treg cells as described above can be used in combination with antigen (allergen, auto-antigen, allo-antigen) to induce the differentiation of antigen-specific Tr1 and/or Th3 cells. This strategy leads to even stronger suppression of Th1- or Th2 mediated immune responses. Moreover, induction of antigen-specific Tr1 cells will lead to long-term immunosuppressive effects in an antigen-specific fashion. Preferably, said allergen is an allergen involved in the induction and/or maintenance of an allergic disease, especially asthma and hence the invention provides a method for inducing and/or increasing tolerance to an allergen involved in asthma. Allergens for use in a method according to the invention include, but are not limited to the list available on the World-Wide Web at http://www.allergen.org/List.htm. Typical example of allergens that result in asthma symptoms are small particles, like house-dust or pollen.

In yet another preferred embodiment, the invention provides a method for inducing and/or increasing tolerance to an allergen in a subject, comprising providing, in the presence of said allergen, said subject with an inhibitor capable of at least in part preventing activation of GITR, wherein said method further comprises providing said subject with a compound capable of activating a Toll-like receptor. Preferably, said Toll-like receptor (TLR) is TLR4 and/or 5 and/or 7 and/or 8. The immunosuppressive effects of CD4⁺CD25⁺ Treg cells are further potentiated by activation of specific Toll-like receptors. CD4⁺CD25⁺ Treg cells in normal naive mice have been shown to selectively express TLR4, 5, 7 and 8, whereas TLR1, 2 and 6 are more broadly expressed on all CD4⁺ T cells (Caramalho *et al.,* 2003). Different TLRs are activated by different microbial products. Ligation of TLR-4 by the ligand lipopolysaccharide strongly increased the suppressor activity of Treg cells (Caramalho *et al.,* 2003). However, TLRs are also expressed on APCs and ligation of these receptors can trigger their maturation, which can enhance the induction and activation of Th1 or Th2 responses. Moreover, the suppressor function of Treg cells can be overridden by activation TLR4 or TLR9 ligands on APCs leading to the production of IL-6 (Pasare *et al.,* 2003). Therefore, targeting of selective TLR ligands (in particular ligands for TLR4, 5, 7 and 8) to Treg cells but not to APCs can be used to increase the immunosuppressive effects of Treg cells.

In another embodiment, the invention provides a method for inducing and/or increasing tolerance to an allergen in a subject, comprising providing, in the presence of said allergen, said subject with an inhibitor capable of at least in part preventing activation of GITR, further providing said subject with a compound capable of inhibiting activation of an antigen presenting cell. Another strategy to selectively stimulate Treg function by TLR ligands without potentiating APC function is the inhibition of APC maturation by a compound that inhibits NF-κB and/or MAPK signal transduction pathways or by blocking of IL-6, its production, or its cell-surface receptor at the time of treatment with TLR ligands. Said NF-κB transducing pathway can for example be inhibited by a compound chosen from the group of anti-oxidant compounds and/or proteasome and/or protease inhibitors, IκB phosphorylation and/or degradation inhibitors, and/or a functional analogue thereof, or by a compound chosen from the group of anti-inflammatory compounds, and/or by a compound chosen from the group of glucocorticosteroid compounds, and/or by a compound chosen from the group of di-hydroxyvitamin D3 compounds and/or a functional analogue thereof, or by a compound from the group of cAMP elevating compounds, and/or by NF-κB decoy- or anti-sense oligonucleotides. Said MAPK/AP-1 signal transducing pathway can for example be inhibited by a compound chosen from the group of non-steroidal anti-inflammatory compounds and/or a functional analogue thereof, and/or by a ligand to a peroxisome proliferator-activated receptor and/or a functional analogue thereof, and/or by a compound of the group of pyridinylimidazole compounds and/or a functional analogue thereof, and/or AP-1 decoy- or anti-sense oligonucleotides.

However, it is also possible to further potentiate the immunosuppressive effects of CD4⁺CD25⁺ Treg cells by activation of the cell-surface receptor CTLA4, which is constitutively expressed by these cells but not by resting CD4⁺CD25- T-lymphocytes. CTLA4 is a member of the of T-cell costimulatory molecules that can provide a negative signal for T-cell activation. The endogenous ligands for CTLA4 are CD80 and CD86 that can be expressed on antigen-presenting cells, in particular mature dendritic cells. However, CD28 that is constitutively expressed by all T-cells is also ligated by CD80 and CD86, leading to a costimulatory signal for T-cells activation upon simultaneous stimulation through their T cell receptor. Since CTLA4 is constitutively present on CD4⁺CD25⁺ Treg cells but not on resting CD4⁺CD25-T cells, ligands that activate this receptor selectively potentiate the immunosuppressive function of Treg cells. Activation of CTLA4 is for example accomplished by a selective monoclonal antibody that is able to activate the receptor and its down-stream signaling pathway. In addition, compounds that have a selective agonistic effect on CTLA4 can also be used for this purpose.

At present, glucocorticoids are used in the treatment of asthma. There is a tendency to at least decrease the amount of used glucocorticoids or preferably, due to its non-selective action, to find an alternative treatment. The invention also provides a method to reduce the amount of GITR in a treatment of allergic diseases. GITR has been discovered by treatment of a mouse T-cell hybridoma with glucocorticoids. Interestingly, glucocorticoids are widely used for the treatment of allergies and asthma. It is anticipated that glucocorticoids interfere with the protective role of naturally occurring Treg cells by increasing GITR expression and hence making these cells more sensitive to inhibition by GITRL. Treatment with inhibitors that inhibit the activation of GITR or its down-stream signaling pathway act synergistically with glucocorticoids for the treatment of allergic diseases, including asthma and other chronic inflammatory diseases such as auto-immune diseases. There is a strong need for so-called "add-on" therapies to limit the use of high doses of glucocorticoids and the associated side-effects. The present invention provides with a combination treatment of glucocorticoids and a compound capable of at least in part preventing activation of GITR, such an "add-on" treatment.

In yet another embodiment the invention provides a method for obtaining a compound capable of at least in part preventing activation of GITR, said method comprising the steps of
- incubating a GITR protein or a functional equivalent and/or a functional fragment thereof with a candidate compound
- determining whether said candidate compound binds to said GITR protein or a functional equivalent and/or a functional fragment thereof
- determining whether said candidate compound blocks or mimics an effect mediated by a GITR-GITRL interaction
- selecting a compound that blocks an effect mediated by a GITR-GITRL interaction.

In a preferred embodiment said method further comprises testing said compound in a non-human animal with features associated with an allergic disease. Even more preferably, said allergic disease is asthma and said non-human animal is a mouse.

A GITR protein or a functional equivalent and/or a functional fragment thereof is for example obtained by isolation of GITR protein from cells naturally expressing GITR protein or from cells (mammalian or bacterial or insect) transfected with GITR cDNA and expressing GITR protein. A functional equivalent and/or a functional fragment of a GITR protein is a protein which is capable of performing essentially the same function as the GITR protein, but not necessarily in the same amount. Typically said equivalent and/or fragment must be capable of binding an (endogenous) GITRL and preferably also capable of activating the downstream signalling pathway. Besides using (partially) purified GITR protein it is also possible to provide said GITR protein as a (cell) membrane-GITR protein complex.

GITR protein or the active part involved in binding to GITRL or GITR expressing cells or cell-membrane preparations derived from these cells are used for screening compounds that bind selectively and with high affinity to this receptor. Examples of such compounds are antibodies or functional fragments thereof, variants (one or more amino acid substitutions) of GITRL or variants of the part involved in binding to GITR, peptides based on GITRL pharmacophore or compounds can be present in random or focussed compound libraries. Screening is e.g. carried out using so-called competition- or displacement binding assays consisting of solubilized GITR protein or the active part involved in binding to GITRL or GITR expressing cells or cell-membrane preparations derived from these cells and a constant amount of labelled (radioactive, fluorescent) ligand (i.e. GITRL or the part involved in binding to GITR). Compounds that bind with sufficient affinity to GITR will compete with ligand binding to GITR and are lead inhibitors. To determine the binding affinity of the lead inhibitor to GITR, several technologies are available such as (radio-)ligand receptor binding assays or scintillation proximity assay or surface plasmon resonance.

Compounds with sufficient affinity for GITR are then, for example, screened in cell-based functional assays whether they block (antagonist) or mimic (agonist) a biological effect mediated by GITR:GITRL interaction in a cell expressing GITR protein. Primary cells expressing GITR isolated from humans or animals or a cell-line expressing GITR or a transfected cell-line (i.e. HEK293) expressing GITR and responding to the activation of GITR by an agonist (i.e. an activating antibody, GITRL) are used to determine the (ant)agonistic capacity of the compounds. Whether a cell is responding is determined directly by measuring a relevant (biological) response such as the activation of the signal transduction pathway (leading to NF-κB activation) or protection from apoptosis or by using a reporter gene (i.e. luciferase) construct or indirectly by using a bioassay with cells of which the biological function (i.e. proliferation) is sensitive to inhibition by the GITR expressing cell.

Compounds that are capable to block the activation of GITR by GITRL are further screened in a non-human animal model with features associated with an allergic disease or in a non-human animal model in which naturally occurring Treg cells are involved to test their *in vivo* capability.

It is clear that this method for obtaining a compound capable of at least in part preventing activation of GITR, can also be based on GITRL. Said method comprises the steps of
- incubating a GITRL protein or a functional equivalent and/or a functional fragment thereof with a candidate compound
- determining whether said candidate compound binds to said GITRL protein or a functional equivalent and/or a functional fragment thereof
- determining whether said candidate compound blocks or mimics an effect mediated by a GITR-GITRL interaction
- selecting a compound that blocks an effect mediated by a GITR-GITRL interaction.

In a further embodiment, the invention provides an isolated, recombinant or synthetic compound obtainable according to the method of the invention. Preferably, said isolated, recombinant or synthetic compound is a proteinaceous substance, like an antibody or a functional equivalent and/or a functional fragment thereof or a peptide.

In yet another embodiment, the invention provides a nucleic acid encoding a proteinaceous compound capable of at least in part preventing activation of GITR. In a preferred embodiment said nucleic acid is part of a vector and more preferably the invention provides a gene delivery vehicle comprising said nucleic acid or said vector.

By equipping a gene delivery vehicle with a nucleic acid molecule encoding a compound capable of at least in part preventing activation of GITR and by targeting said vehicle in a subject to a site where symptoms associated with the presence of an allergen are present, said gene delivery vehicle provides said subject with the necessary means of preventing activation of GITR and to subsequent suppression of Th2-mediated allergic disorders. Such a gene delivery vehicle, which is an independently infectious vector may for example be a virus (like an adenovirus or a retrovirus), or a liposome, or a polymer, or the like, that in it self can infect or in any other way deliver genetic information to a preferred site of treatment. Furthermore, the invention provides a gene delivery vehicle which has additionally been supplemented with a specific ligand or target molecule or target molecules, by which the gene delivery vehicle can be specifically directed to deliver its genetic information at a target cell of choice. Such a target molecule may for instance be a receptor molecule.

In yet another embodiment the invention provides a pharmaceutical composition comprising an isolated, recombinant or synthetic compound capable of at least in part preventing activation of GITR, a nucleic acid encoding a proteinaceous compound, a vector or a gene delivery vehicle.

A pharmaceutical composition can either be in a solid form (for example, a pill) or in a fluidised form (for example, a liquid formulation). It is clear to a person skilled in the art that the active ingredient (for example, a proteinaceous substance capable of at least in part preventing activation of GITR) can be accompanied by a pharmaceutical acceptable carrier or diluent. Such a pharmaceutical can be applied via different routes, for example via a local injection, dermal application, intravenously or via inhalation. It is clear to a person skilled in the art that said pharmaceutical can further comprise a compound capable of activating a Toll-like receptor and/or a compound capable of inhibiting activation of an antigen presenting cell and/or a compound that has a selective agonistic effect on CTLA4. In a preferred embodiment a pharmaceutical composition according to the invention further comprises an allergen, more preferably said allergen is an allergen involved in allergic disease and even more preferably said allergic disease is asthma.

The invention furthermore provides use of an isolated, recombinant or synthetic compound capable of at least in part preventing activation of GITR, a nucleic acid encoding said proteinaceous compound, a vector or a gene delivery vehicle for the preparation of a medicament for the treatment of an allergy, for example asthma. Preferably, said compound is an inhibitor of GITR or said compound at least in part inhibits the expression of GITR or said compound is an inhibitor of a GITRL or said compound at least in part inhibits the expression of GITRL.

In yet another embodiment the invention provides a method for inducing and/or increasing tolerance to an allergen in a subject, comprising increasing in said subject in which said allergen is present the number of CD4⁺CD25⁺GITR⁺ T cells at a site of allergic disease. The present invention discloses the immunosuppressive function of Treg cells with regard to symptoms induced and/or maintained by an allergen and hence increasing the number of Treg cells (CD4⁺CD25⁺GITR⁺ T cells) results in an increase in immunosuppression. To increase the number of Treg cells at site of immune- or inflammatory responses, local administration of chemokines that are involved in the recruitment of Treg cells are used. Chemokines and adhesion molecules regulate the migration of functionally different T-cells to lymphoid- or peripheral tissues. CD4⁺CD25⁺ Treg cells respond to several inflammatory and lymphoid chemokines in particular the chemokines CCL1 (I-309), CCL4 (MIP-1β), CCL17 (TARC), CCL22 (MDC) (Bystry *et al.,* 2001; Colantonio *et al.,* 2002; Iellem *et al.,* 2001). In agreement herewith, CD4⁺CD25⁺ Treg cells specifically express the chemokine receptors CCR4, CCR5 and CCR8 (Bystry *et al.,* 2001; Iellem *et al.,* 2001). Importantly, APCs can produce these kind of chemokines upon activation or maturation and subsequently can attract CD4⁺CD25⁺ Treg cells to sites of inflammation or immune responses (Bystry *et al.,* 2001; Iellem *et al.,* 2001). However, these pathways for recruitment to sites of inflammation are not unique for Treg cells since these receptors are also expressed by Th1 and Th2 lymphocytes. Thus, to increase the number of Treg cells in Th1-dominated inflammatory responses, local administration (i.e. affected joint in case of arthritis) of chemokines that activate CCR8 and/or CCR4 will be effective. This may be accomplished by agonists including but not limited to I-309, TARC and MDC or functional analogues (small molecules, peptides) of these chemokines. To increase the number of Treg cells in Th2-dominated inflammatory responses, local administration (i.e. inhalation in case of asthma) of chemokines that activate CCR5 will be effective. This may be accomplished by agonists including but not limited to MIP-1β, MIP-1α, RANTES or functional analogues (small molecules, peptides) of these chemokines.

Preferably, said increase in the number of Treg cells is obtained by providing said subject with chemokines or a functional fragment and/or a functional analogue thereof and even more preferably said chemokines are locally administered. Depending on which T cells are involved the immune response to an allergen, the following options can be applied. When said allergic response is a Th1-dominated response, said chemokine or a functional fragment and/or a functional analogue thereof is capable of activating CCR8 and/or CCR4. Examples of chemokines that can be applied in a Th1-dominated response are 1-309, TARC or MDC or a functional fragment and/or a functional analogue thereof. However, when said allergic response is a Th2-dominated response, said chemokine or a functional fragment and/or a functional analogue thereof is capable of activating CCR5. Examples of chemokines that can be applied in a Th2-dominated response are MIP-1β, MIP-1α or RANTES or a functional fragment and/or a functional analogue thereof.

It is clear that this method that involves increasing the number of Treg cells can be combined with any other method, as described herein, for increasing the immunosuppressive capability of Treg cells, for example by combining with a compound capable of at least in part preventing activation of GITR and/or a compound capable of activating a Toll-like receptor and/or a compound capable of inhibiting activation of an antigen presenting cell and/or a compound capable of activating CTLA4.

In yet another embodiment, the invention provides a method for suppressing GITR activation of cells expressing said GITR, comprising providing, in the presence of an allergen, said cells with an inhibitor of GITR. Said method is easily performed *in vitro*, and hence said method is preferably used in screening assays.

The invention will be explained in more detail in the following description, which is not limiting the invention.

### EXPERIMENTAL PART

### MATERIALS AND METHODS

**Animals**. Animal care and use were performed in accordance with the guidelines of the Dutch Committee of Animal Experiments. Specific pathogen-free male BALB/c mice (6-8 wk old) were purchased from Charles River (Maastricht, The Netherlands) and housed in macrolon cages in a laminar flow cabinet and provided with food and water *ad libitum.*

**Sensitization, treatment and challenge.** Mice were sensitized intraperitoneally (i.p.) on days 0 and 7 with 10 µg OVA (grade V, Sigma-Aldrich) in 0.1 ml alum (Pierce, Rockford, Illinois). After two weeks, sensitized mice were exposed to three OVA inhalation challenges (10 mg/ml in saline) for 20 min every third day. In some experiments, mice were treated by i.p. injection of 1 mg rat monoclonal antibody to mouse GITR (DTA-1) or control antibody (rat IgG) prior to OVA inhalation challenge.

### Isolation of lung cells for flow cytometry and restimulation in vitro.

Mice were sacrificed by i.p. injection of 1 ml 10% urethane (Sigma-Aldrich) in saline and lungs were perfused via the right ventricle with 4 ml saline containing 100 U/ml heparin (Leo Pharmaceuticals, Weesp, Netherlands). Lungs were perfused via the right ventricle with 4 ml saline containing 100 U/ml heparin (Leo Pharmaceuticals, Weesp, The Netherlands). Next, the thoracic lymph nodes (TLN), derived from the paratracheal and parabronchial regions, and the lungs were removed. Subsequently, single-cell suspension from lungs and TLNs were incubated with Ab against FcγRII/III (24G2) to block aspecific binding. To determine the number of CD4⁺CD25⁺CD45RB^{Io} Treg cells, cell suspensions were stained with Cy-ChromeTM-conjugated anti-CD4 (clone H129.19), PE-conjugated anti-CD25 (clone PC61) and FITC-conjugated anti-CD45RB (clone 16A). After staining, cells were analyzed with FACScanTM flow cytometer (Becton Dickinson) using the CELLQuestTM software program.
For T-lymphocyte restimulation, lung cells were suspended in culture medium (RPMI 1640 enriched with 10 % FCS, 1 % glutamax I, gentamicin (all from Life Technologies, Gaithersburg, Maryland), and 50 µM β-mercaptoethanol (Sigma-Aldrich, Zwijndrecht, The Netherlands)) at a concentration of 6x10⁵ cells/well in round-bottom 96-well plates (Greiner Bio-One GmbH, Kremsmuenster, Austria) in the absence or presence of plate-bound rat antimouse CD3 mAb (clone 17A2, 50 µg/ml, coated 16 h at 4°C). Each stimulation was performed in triplicate. After 5 days of culture at 37°C, the supernatants were harvested, pooled per stimulation, and stored at -20°C until cytokine levels were determined by ELISA.

Cytokine ELISAs. IL-5, IL-10 and IL-13 (BD PharMingen) ELISAs were performed according to the manufacturer's instructions. The detection limits of the ELISAs were 32 pg/ml for IL-5, 15 pg/ml for IL-10 and 15 pg/ml for IL-13.

**Measurement of airway responsiveness in *vivo.*** Twenty-four hours after the last OVA challenge, airway responsiveness was measured in conscious, unrestrained mice using barometric whole-body plethysmography by recording respiratory pressure curves (Buxco, EMKA Technologies, Paris, France) in response to inhaled methacholine (acetyl-β-methylcholine chloride, Sigma-Aldrich). Airway responsiveness was expressed in enhanced pause (Penh), as described in detail previously (Deurloo *et al.,* 2001). Briefly, mice were placed in a whole-body chamber and basal readings were determined for 3 min. Aerosolized saline, followed by doubling concentrations of methacholine (ranging from 1.6-25 mg/ml in saline), were nebulized for 3 min, and readings were determined for 3 min after each nebulization. The effective dose of methacholine that induced a half-maximal response, the ED₅₀ value, was calculated after correction for baseline Penh values.

**Determination of OVA-specific immunoglobulin levels in serum.** After measurement of airway responsiveness in vivo, mice were sacrificed by i.p. injection of 1 ml 10% urethane in saline and were bled by cardiac puncture. Subsequently, serum was collected and stored at -70°C until analysis. Serum levels of OVA-specific IgE were measured by sandwich ELISA as described previously (Deurloo *et al.,* 2001).

**Statistical analysis.** All data are expressed as mean ± standard error of mean (SEM). The airway dose-response curves to methacholine were statistically analyzed by a general linear model of repeated measurements followed by post-hoc comparison between groups. Data were log transformed before analysis to equalize variances in all groups. All other data were analyzed using a Student's t test (2-tailed, homosedastic). Results were considered statistically significant at the p<0.05 level.

### EXPERIMENTAL PART

### RESULTS

### Example 1.

**The number of naturally occurring professional Treg cells is increased in lungs obtained from a mouse model of allergic asthma**

We examined whether the number of naturally occurring professional Treg cells changes upon allergen inhalation in a mouse model of allergic asthma. Balb/c mice were sensitized by the model allergen ovalbumin in the presence of the adjuvant alum as described previously (Deurloo *et al.,* 2001). Upon repeated allergen inhalation challenge, mice develop airway manifestations of allergic asthma such as airway hyperresponsiveness to methacholine and eosinophilic airway inflammation (Deurloo *et al.,* 2001). To determine the number of professional Treg cells, lung tissue cells and lung-draining lymph node cells were isolated 24 hours after the last challenge, as described previously (Deurloo *et al.,* 2001) and stained using monoclonal antibodies to specific cell-surface molecules CD4, CD25 and CD45RB. The percentage of CD25⁺CD45RB^{Io} of all CD4⁺ T-lymphocytes in lung tissue or lung-draining lymph nodes was determined by flow cytometry. The number of CD4⁺CD25⁺CD45RB^{1o} cells in lung tissue from ovalbumin challenged mice (n=3) was significantly (P<0.01, student's t-test) increased as compared to the number in lung tissue from control mice (n=4) challenged with saline (21.75 ± 0.63 versus 9.25 ± 0.93, respectively). In contrast, the number of CD4⁺CD25⁺CD45RB^{Io} cells in lung-draining lymph nodes were not different between ovalbumin and saline-challenged mice (6.19 ± 1.21 versus 5.49 ± 1.01, respectively).

These data clearly demonstrate that upon allergen inhalation by previously sensitized mice, the number of professional Treg cells (CD4⁺CD25⁺CD45RB^{Io}) increases specifically in the affected organ, the lung.

### Example 2.

**Inactivation of Treg cells by an activating monoclonal antibody to GITR potentiates allergen-induced airway manifestations of asthma**

We examined whether inactivation of professional Treg cells by an activating antibody to GITR was able to affect the induction of airway manifestations of asthma in a mouse model of allergic asthma. Balb/c mice were sensitized by ovalbumin in alum adjuvant as described previously (Deurloo *et al.,* 2001). Twenty days after sensitization, blood samples were obtained to determine serum IgE antibody levels to ovalbumin. Subsequently, the airway responsiveness to the bronchospasmogenic stimulus methacholine was determined by whole-body plethysmography as described previously (Deurloo *et al.,* 2001). Prior to the first ovalbumin inhalation challenge, mice were divided into two groups of 6 animals: one group received and intraperitoneal injection 1 mg of endotoxin-free monoclonal antibody to GITR (DTA-1); the other group received 1 mg rat IgG as control treatment. Subsequently, mice were challenged three times (once a day, every third day) by inhalation of ovalbumin (10 mg/ml). Twenty-four hours after the last inhalation challenge, airway responsiveness to methacholine was again determined (Deurloo *et al.,* 2001).

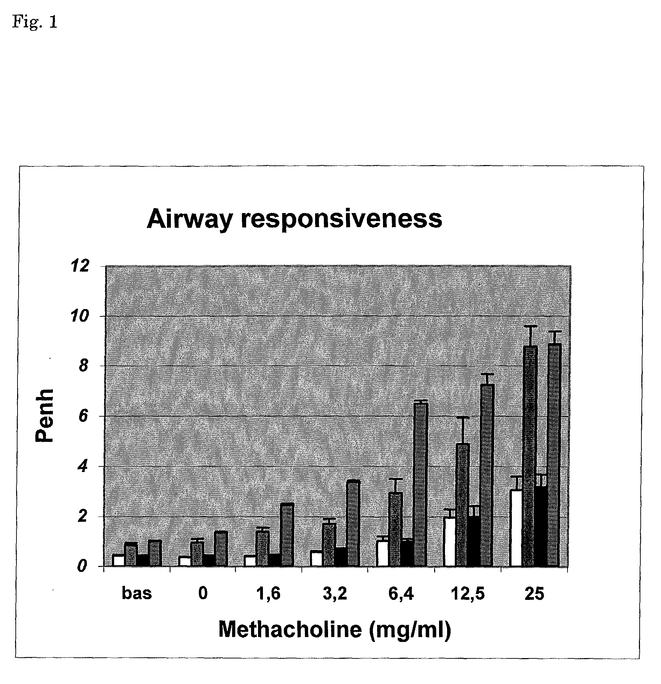

In mice treated with treated with control antibody, serum levels of ovalbumin specific IgE were significantly increased after ovalbumin challenge as compared to pre-challenge values (Table 1). Interestingly, treatment with anti-GITR strongly potentiated (P<0.01, student's t-test) the upregulation of serum ovalbumin-specific IgE levels after ovalbumin inhalation challenge (Table 1). Furthermore, in mice treated with treated with control antibody, the airway responsiveness to methacholine was significantly increased after ovalbumin challenge as compared to pre-challenge values (figure 1). Treatment with anti-GITR strongly potentiated (P<0.01) the allergen-induced airway hyperresponsiveness to methacholine (figure 1). Moreover, a leftward shift of the dose-response curve, a cardinal feature of severe asthma, was observed in ovalbumin-challenged mice treated with anti-GITR as compared to control antibody treated mice. The ED₅₀ value of the methacholine dose-response curve in ovalbumin challenged mice was significantly (P<0.01) decreased from 12.6 ± 1.5 mg/ml methacholine after treatment with control antibody to 5.5 ± 1.3 mg/ml in mice treated with anti-GITR.

It is concluded that inactivation of naturally occurring professional Treg cells by treatment with an activating antibody to GITR strongly potentiates allergen induced airway hyperresponsiveness and IgE upregulation in this mouse model of asthma.

Table 1. Serum levels of ovalbumin-specific IgE (Units/ml). Ovalbumin sensitized BALB/c mice (n=6 per group) were treated with control antibody (Control) or anti-GITR prior to repeated ovalbumin inhalation challenges. Levels of ovalbumin-specific IgE in serum were measured before and after ovalbumin inhalation challenges. *: P<0.01 as determined by student's t-test and compared to the levels before ovalbumin inhalation challenge. #: P<0.01 as determined by student's t-test and compared to control antibody treated mice.

| **Treatment** | **Before** | **After** |
|---|---|---|
| Control | 4,988 ± 1,788 | 87,581 ± 24,189* |
| Anti-GITR | 7,373 ± 1,806 | 321,380 ± 94,270*# |

### Example 3.

**Inactivation of Treg cells by an activating monoclonal antibody to GITR potentiates Th2 type cytokine production.**

Next, we examined the cytokine production upon anti-CD3 restimulation of lung lymphocytes obtained from ovalbumin sensitized and challenged mice treated *in vivo* with control antibody or anti-GITR (see example 2). Lymphocytes were isolated from ovalbumin sensitized and challenged mice and restimulated *in vitro* as described previously (Deurloo *et al.,* 2001). Lung lymphocyte cultures from animals treated *in vivo* with anti-GITR during antigen inhalation challenge, produced significantly (P<0.05, student's t-test) potentiated amounts of the Th2 type cytokines IL5, IL10 and IL13 as compared to mice treated with control antibody (Table 2).

It is concluded that inactivation of naturally occurring professional Treg cells by treatment with an activating antibody to GITR potentiates Th2 type cytokine production.

Table 2. Cytokine production by lung lymphocytes obtained from ovalbumin sensitized and challenged BALB/c mice treated *in vivo* with control antibody (control) or anti-GITR. Lung lymphocyte cultures were restimulated with plate-bound anti-CD3 for five days. *: P<0.05 and **: P<0.01 as determined by student's t-test and compared to control antibody treated mice.

| **Treatment** | **IL5 (pg/ml)** | **IL10 (pg/ml)** | **IL13 (pg/ml)** |
|---|---|---|---|
| Control | 15,872 ± 6,451 | 285 ± 37 | 2,942 ± 736 |
| Anti-GITR | 39,100 ± 4,346* | 1,046 ± 226** | 11,653 ± 1,670** |

### DESCRIPTION OF FIGURES

Figure 1. Airway responsiveness to inhalation of different doses of methacholine. Ovalbumin sensitized BALB/c mice (n=6 per group) were treated with control antibody (open and hatched bars) or anti-GITR (black and cross-hatched bars) prior repeated ovalbumin inhalation challenges. Airway responsiveness was measured before (black and white bars) and after (hatched and crosshatched bars) ovalbumin inhalation challenges. The methacholine dose-response curves after ovalbumin challenge are significantly (P<0.01) different from those before challenge. The methacholine dose-response curve after ovalbumin challenge of mice treated with anti-GITR is significantly (P<0.01) different from the curve after ovalbumin challenge of mice treated with control antibody.

### REFERENCES

Bystry, R.S., Aluvihare, V., Welch, K.A., Kallikourdis, M. & Betz, A.G. (2001). B cells and professional APCs recruit regulatory T cells via CCL4. Nat Immunol, 2, 1126-32.

Caramalho, I., Lopes-Carvalho, T., Ostler, D., Zelenay, S., Haury, M. & Demengeot, J. (2003). Regulatory T cells selectively express Toll-like receptors and are activated by lipopolysaccharide. J Exp Med, 197, 403-11.

Colantonio, L., Iellem, A., Sinigaglia, F. & D'Ambrosio, D. (2002). Skin-homing CLA+ T cells and regulatory CD25+ T cells represent major subsets of human peripheral blood memory T cells migrating in response to CCL1/I-309. Eur J Immunol, 32, 3506-14.

Deurloo, D.T., van Esch, B.C., Hofstra, C.L., Nijkamp, F.P. & van Oosterhout, A.J. (2001). CTLA4-IgG reverses asthma manifestations in a mild but not in a more "severe" ongoing murine model. Am J Respir Cell Mol Biol, 25, 751-60.

Dieckmann, D., Bruett, C.H., Ploettner, H., Lutz, M.B. & Schuler, G. (2002). Human CD4(+)CD25(+) Regulatory, Contact-dependent T Cells Induce Interleukin 10-producing, Contact-independent Type 1-like Regulatory T Cells. J Exp Med, 196, 247-53.

Iellem, A., Mariani, M., Lang, R., Recalde, H., Panina-Bordignon, P., Sinigaglia, F. & D'Ambrosio, D. (2001). Unique chemotactic response profile and specific expression of chemokine receptors CCR4 and CCR8 by CD4(+)CD25(+) regulatory t cells. J Exp Med, 194, 847-54.

Jonuleit, H., Schmitt, E., Kakirman, H., Stassen, M., Knop, J. & Enk, A.H. (2002). Infectious Tolerance: Human CD25(+) Regulatory T Cells Convey Suppressor Activity to Conventional CD4(+) T Helper Cells. J Exp Med, 196, 255-60.

Jonuleit, H., Schmitt, E., Schuler, G., Knop, J. & Enk, A.H. (2000). Induction of Interleukin 10-producing, Nonproliferating CD4(+) T Cells with Regulatory Properties by Repetitive Stimulation with Allogeneic Immature Human Dendritic Cells. J Exp Med, 192, 1213-1222.

Jonuleit, H., Schmitt, E., Stassen, M., Tuettenberg, A., Knop, J. & Enk, A.H. (2001a). Identification and functional characterization of human cd4(+)cd25(+) t cells with regulatory properties isolated from peripheral blood. J Exp Med, 193, 1285-94.

McHugh, R.S., Whitters, M.J., Piccirillo, C.A., Young, D.A., Shevach, E.M., Collins, M. & Byrne, M.C. (2002). CD4(+)CD25(+) Immunoregulatory T Cells. Gene expression analysis reveals a functional role for the glucocorticoid-induced TNF receptor. Immunity, 16, 311-23.

Pasare, C. & Medzhitov, R. (2003). Toll Pathway-Dependent Blockade of CD4+CD25+ T Cell-Mediated Suppression by Dendritic Cells. Science, 16, 16.

Roncarolo, M. & Levings, M.K. (2000). The role of different subsets of T regulatory cells in controlling autoimmunity. Curr Opin Immunol, 12, 676-83.

Shevach, E.M. (2002). CD4+ CD25+ suppressor T cells: more questions than answers. Nat Rev Immunol, 2, 389-400.

Shimizu, J., Yamazaki, S., Takahashi, T., Ishida, Y. & Sakaguchi, S. (2002). Stimulation of CD25+CD4+ regulatory T cells through GITR breaks immunological self-tolerance. Nat Immunol, 3, 135-142.

Suto, A., Nakajima, H., Kagami, S.I., Suzuki, K., Saito, Y. & Iwamoto, I. (2001). Role of cd4(+) cd25(+) regulatory t cells in t helper 2 cell-mediated allergic inflammation in the airways. Am J Respir Crit Care Med, 164, 680-7.

Thornton, A.M. & Shevach, E.M. (2000). Suppressor effector function of CD4+CD25+ immunoregulatory T cells is antigen nonspecific. J Immunol, 164, 183-90.

Zuany-Amorim, C., Sawicka, E., Manlius, C., Le Moine, A., Brunet, L.R., Kemeny, D.M., Bowen, G., Rook, G. & Walker, C. (2002). Suppression of airway eosinophilia by killed Mycobacterium vaccae-induced allergen-specific regulatory T-cells. Nat Med, 8, 625-9.

## Claims

1. A method for regulating tolerance to an allergen in a subject, comprising providing, in the presence of said allergen, said subject with an inhibitor capable of regulating the activation of a glucocorticoid-induced tumor necrosis factor receptor (GITR).

2. A method for inducing and/or increasing tolerance to an allergen in a subject, comprising providing, in the presence of said allergen, said subject with an inhibitor capable of at least in part preventing activation of GITR.

3. A method according to claim 2, wherein said inhibitor at least in part prevents ligation of GITR by an endogenous GITR-ligand (GITRL).

4. A method according to any one of claims 1 to 3, wherein said GITR is present on a CD4⁺CD25⁺ regulatory T cell.

5. A method according to any one of claims 1 to 4, wherein said inhibitor is an inhibitor of GITR.

6. A method according to any one of claims 1 to 4, wherein said inhibitor at least in part inhibits the expression of GITR.

7. A method according to any one of claims 1 to 4, wherein said inhibitor is an inhibitor of a GITRL.

8. A method according to any one of claims 1 to 4, wherein said inhibitor at least in part inhibits the expression of GITRL.

9. A method according to any one of claims 1 to 8, wherein said subject is further provided with said allergen.

10. A method according to any one of claims 1 to 9, wherein said allergen is an allergen involved in allergic disease.

11. A method according to claim 10, wherein allergic disease is asthma.

12. A method according to any one of claims 1 to 11, further comprising providing said subject with a compound capable of activating a Toll-like receptor.

13. A method according to any one of claims 1 to 12, further comprising providing said subject with a compound capable of inhibiting activation of an antigen presenting cell.

14. A method for obtaining a compound capable of at least in part preventing activation of GITR, said method comprising the steps of
- incubating a GITR protein or a functional equivalent and/or a functional fragment thereof with a candidate compound
- determining whether said candidate compound binds to said GITR protein or a functional equivalent and/or a functional fragment thereof
- determining whether said candidate compound blocks or mimics an effect mediated by a GITR-GITRL interaction
- selecting a compound that blocks an effect mediated by a GITR-GITRL interaction.

15. A method according to claim 14, further comprises testing said compound in a non-human animal with features reminiscent of an allergic disease.

16. An isolated, recombinant or synthetic compound obtainable according to the method of claim 14 or 15.

17. An isolated, recombinant or synthetic compound according to claim 16, wherein said compound is a proteinaceous substance.

18. An isolated, recombinant or synthetic compound according to claim 17, wherein said proteinaceous substance is an antibody or a functional equivalent and/or a functional fragment thereof.

19. A nucleic acid encoding a compound according to claim 17 or 18.

20. A vector comprising a nucleic acid according to claim 19.

21. A gene delivery vehicle comprising a vector according to claims 20.

22. A pharmaceutical composition comprising an isolated, recombinant or synthetic compound according to anyone of claims 16 to 18, a nucleic acid according to claim 19, a vector according to claim 20 or a gene delivery vehicle according to claim 21.

23. A pharmaceutical composition according to claim 22, further comprising an allergen.

24. A pharmaceutical composition according to claim 23, wherein said allergen is an allergen involved in allergic disease.

25. A pharmaceutical composition according to claim 24, wherein said allergic disease is asthma.

26. Use of an isolated, recombinant or synthetic compound according to anyone of claims 16 to 18, a nucleic acid according to claim 19, a vector according to claim 20 or a gene delivery vehicle according to claim 21 for the preparation of a medicament for the treatment of an allergy.

27. Use according to claim 26 wherein said compound is an inhibitor of GITR or wherein said compound at least in part inhibits the expression of GITR or wherein said compound is an inhibitor of a GITRL or wherein said compound at least in part inhibits the expression of GITRL.

28. Use according to claim 26 or 27, wherein said allergy is asthma.

29. A method according to any one of claims 1 to 13, further comprising providing said subject with a compound capable of activating a CTLA4 receptor.

30. A method for suppressing GITR activation of cells expressing said GITR, comprising providing, in the presence of an allergen, said cells with an inhibitor of GITR.
